# DEMANDE DE BREVET EUROPEEN

(11) **EP 3 338 739 A1**
(43) Date de publication de la demande: **27.06.2018**
(21) Numéro de dépôt: 17209203.3
(22) Date de dépôt: 21.12.2017
(51) Int. Cl.: A61F 2/38

(54) **PARTIE FÉMORALE D'UNE PROTHÈSE DE GENOU**

(30) Priorité: 26.12.2016 FR 1670786
(71) Demandeur: X.NOV IP, 1882 Luxembourg (LU)
(72) Inventeur: BIEGUN, Frédérique, 2926 Boncourt (CH); BIEGUN, Jean-François, 2926 Boncourt (CH); MARCEAUX, Pascal, 52000 Chaumont (FR); LOEHLE, Pascal, 2904 Bressaucourt (CH)
(74) Mandataire: Eidelsberg, Olivier Nathan

(57) **Abrégé**

Partie (3) fémorale, comportant au moins un condyle, de préférence deux condyles séparés dans la partie postérieure par un espace inter-condyle, et une partie de trochlée, le au moins un condyle, notamment les deux condyles, et la partie de trochlée définissant entre eux un espace en forme de cage (5) comportant des faces destinées à venir en contact avec l'os du fémur, la cage comportant au moins deux faces intérieures, planes ou sensiblement planes, à savoir une face (8) intérieure plane du côté antérieur de la partie la plus proximale du au moins un condyle et une face (10) intérieure plane du côté postérieur de la partie la plus proximale de la partie de trochlée, caractérisée en ce que la face (8) intérieure plane du côté antérieur de la partie la plus proximale du au moins un condyle, notamment du condyle latéral et/ou du condyle médial, est inclinée par rapport à l'axe mécanique du fémur, notamment d'un angle compris entre 1° et 5°, par exemple de 3°, en convergeant avec cet axe dans la direction proximale, et la face intérieure plane du côté antérieur de la partie la plus proximale du au moins un condyle est inclinée par rapport à la face (10) intérieure plane la plus proximale du côté antérieur de la partie de trochlée d'un angle tel que ces deux faces convergent l'une vers l'autre dans la direction distale.

## Description

La présente invention se rapporte à une partie fémorale d'une prothèse de genou, ainsi qu'à une prothèse totale de genou comportant une partie tibiale, un insert ou ménisque et une partie fémorale de ce genre.

Les parties fémorales sont la partie de la prothèse de genou destinée à être solidarisée à l'extrémité distale du fémur et qui comporte généralement du côté postérieur deux condyles, respectivement latéral et médial, et, du côté antérieur, une partie de trochlée dans la surface antérieure de laquelle est définie un chemin de trochlée, la partie fémorale étant fixée à l'extrémité du fémur et venant en contact avec un insert ou ménisque tibial, qui est posé sur un plateau tibial, lui-même ancré à l'extrémité proximale du tibia.

On connaît déjà des parties fémorales, par exemple du document US 8 398 715. Une partie fémorale telle que décrite dans ce document de l'art antérieur est constituée de deux condyles séparés dans la partie postérieure par un espace intercondyle et reliés entre eux dans la partie antérieure par une partie de trochlée. Du côté extérieur, les deux condyles ont une forme, vu en coupe antéro-postérieure ou sagittale, sensiblement circulaire pour venir rouler dans des surfaces concaves respectives formées dans la face supérieure du ménisque et assurer par congruence avec celles ci une flexion relative de la partie fémorale par rapport à la partie tibiale pour ainsi imiter la flexion du genou. Les deux condyles et la partie de trochlée définissent entre eux un espace en forme de cage comportant des faces destinées à venir en contact avec l'os du fémur, préalablement réséqué par le chirurgien pour s'y adapter et y être solidarisé à demeure.

Classiquement, la cage comporte quatre faces intérieures, planes ou sensiblement planes, à savoir une face intérieure plane du côté antérieur de la partie la plus proximale du condyle latéral, une face intérieure plane du côté antérieur de la partie la plus proximale du condyle médial, une face intérieure plane du côté postérieur de la partie la plus proximale de la partie de trochlée et une face de fond, s'étendant entre chaque face intérieure des parties les plus proximales des condyles et la face intérieure de la partie la plus proximale de la partie de trochlée. De manière générale, le positionnement et la fixation de la cage à l'extrémité distale du fémur est difficile à réaliser. Il en résulte souvent une mauvaise solidarisation qui, en raison des contraintes élevées de cisaillement subies par l'articulation du genou, entraîne un mouvement relatif entre l'os du fémur et la partie fémorale, voir un détachement, nécessitant une nouvelle intervention du chirurgien.

On connaît de EP 0 941 719, FR 3 019 032 et EP 1 354 571 des parties fémorales suivant le préambule de la revendication 1.

La présente invention vise à surmonter les inconvénients de l'art antérieur en proposant une partie fémorale d'une prothèse de genou que l'on peut positionner très simplement sur le fémur réséqué et qui a une longue durée de vie, notamment en résistant bien aux efforts de cisaillement tendant à la décoller du fémur.

Suivant l'invention, une partie fémorale, comportant au moins un condyle, de préférence deux condyles séparés par un espace inter-condyle, et une partie de trochlée, le au moins un condyle, notamment les deux condyles, et la partie de trochlée définissant entre eux un espace en forme de cage comportant des faces destinées à venir en contact avec l'os du fémur, la cage comportant au moins deux faces intérieures, planes ou sensiblement planes, à savoir une face intérieure plane du côté antérieur de la partie la plus proximale du au moins un condyle et une face intérieure plane du côté postérieur de la partie la plus proximale de la partie de trochlée, est caractérisée en ce que la face intérieure plane du côté antérieur de la partie la plus proximale du au moins un condyle, notamment du condyle latéral et/ou du condyle médial, est inclinée par rapport à l'axe mécanique du fémur, notamment d'un angle compris entre 1° et 5°, par exemple de 3°, en convergeant avec cet axe dans la direction proximale, et la face intérieure plane du côté antérieur de la partie la plus proximale du au moins un condyle est inclinée par rapport à la face intérieure plane la plus proximale du côté antérieur de la partie de trochlée d'un angle tel que ces deux faces convergent l'une vers l'autre dans la direction distale, c'est-à-dire que les deux faces sont convergentes, en section sagittale, vers un point se trouvant du côté du tibia.

En prévoyant un tel agencement, on améliore fortement l'opposition au descellement, car une partie des contraintes de cisaillement sont transformées en contraintes de compression, notamment au niveau de la partie postérieure. Plus l'effort généré par le tibia sur l'articulation fémur-tibia est important, plus l'os du fémur est comprimé et sollicité et plus la partie fémorale sera susceptible d'avoir un bon ancrage et une bonne ostéointégration.

De préférence, le au moins un condyle comporte deux condyles respectivement médial et latéral, et les faces intérieures des deux condyles sont parallèles.

De préférence, la cage comporte aussi une face intérieure plane de fond, disposée entre la face intérieure plane du côté antérieur de la partie la plus proximale du au moins un condyle et la face intérieure plane la plus proximale du côté antérieur de la partie de trochlée, la face de fond étant perpendiculaire à l'axe mécanique du fémur.

De préférence, l'angle de convergence distale est compris entre 1 et 35°, encore plus préférablement entre 15 et 25° .

A titre d'exemple, on décrit maintenant un mode de réalisation préféré de l'invention en se reportant aux dessins dans lesquels :
- la figure 1: est une vue en coupe sagittale d'une prothèse totale de genou comportant une partie fémorale suivant l'invention ; et
- la figure 2: est une vue en coupe sagittale plus en détail de la partie fémorale de la prothèse représentée à la figure 1.

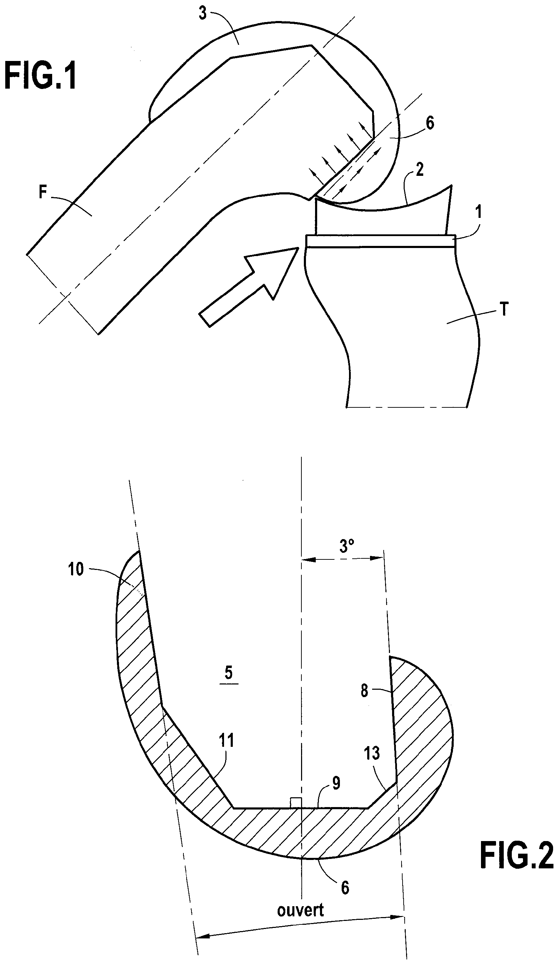

A la figure 1, il est représenté une prothèse de genou positionnée sur un patient. La prothèse de genou comporte un plateau 1 tibial ancré dans le tibia T et supportant sur sa face supérieure un ménisque 2, qui lui-même est en contact avec une partie 3 fémorale fixée au fémur F. Le ménisque 2 comporte à sa face supérieure deux régions concaves qui coopèrent avec des parties 6 de condyle de la partie 3 fémorale.

La partie fémorale est représentée également à la figure 2, sans que soit représenté le fémur. La vue est en coupe sagittale suivant un axe décalé par rapport à l'axe antéro-postérieur au niveau du condyle latéral. La coupe serait identique au niveau du condyle médial. Vu en coupe sagittale, la partie fémorale est constituée du côté postérieur d'une partie formant condyle et du côté antérieur d'une partie de trochlée définissant sur sa surface extérieure (du côté antérieur) un chemin de trochlée destiné à coopérer avec une trochlée. Du côté extérieur (du côté postérieur), la partie formant condyle a, en coupe sagittale, une forme sensiblement circulaire destinée à coopérer avec une forme correspondante de la région concave supérieure correspondante formée sur la surface supérieure du ménisque 2.

Entre les deux parties formant condyles respectivement médial et latéral et la partie de trochlée, la partie 3 fémorale définit une cage 5 intérieure destinée à s'adapter à l'extrémité distale du fémur après résection de ce dernier par le chirurgien. La cage 5 intérieure, vue en coupe sagittale, est délimitée par plusieurs faces sensiblement planes, notamment une face 8 d'extrémité postérieure de condyle, une face 9 plane distale de fond et une face 10 d'extrémité antérieure de partie de trochlée. La face 9 de fond est perpendiculaire à l'axe mécanique du fémur. Les deux faces 8 et 9 sont reliées par une face 13 intermédiaire qui ici est plane mais, dans d'autres modes de réalisation, pourrait être incurvée, notamment en forme d'arc de cercle. De même, la face 9 de fond et la face 10 de trochlée sont reliées par une face 11 intermédiaire qui ici est plane mais, dans d'autres modes de réalisation, pourrait être incurvée, notamment en forme d'arc de cercle. On peut également, dans d'autres modes de réalisation, réaliser une ou plusieurs faces planes supplémentaires entre la face de fond et respectivement la face d'extrémité de trochlée et/ou la ou les faces d'extrémité de condyle.

En outre, dans le mode de réalisation représenté, les faces intérieures des deux condyles sont parallèle. Dans d'autres mode de réalisation, elles pourraient être réalisées de manière différentes, notamment en terme d'inclinaisons relatives.

Le plan défini par la face 8 plane intérieure de chacun des condyles médial et latéral fait un angle avec le plan défini par la face 10 plane intérieure de la partie de trochlée. Ces deux plans sont perpendiculaires au plan antéro-postérieur ou sagittal, et l'angle formé est tel que les deux plans se rejoignent de manière virtuelle du côté de la partie fémorale opposée au fémur, c'est-à-dire distalement ou du côté du tibia.

En outre, le plan défini par la face 8 plane intérieure des deux condyles fait un angle avec le plan défini par l'axe médiolatéral et l'axe mécanique, notamment de 1 à 4°, par exemple de 3°, les deux plans se rejoignant du côté du fémur, c'est-à-dire proximalement.

## Revendications

1. Partie (3) fémorale, comportant au moins un condyle, de préférence deux condyles séparés dans la partie postérieure par un espace inter-condyle, et une partie de trochlée, le au moins un condyle, notamment les deux condyles, et la partie de trochlée définissant entre eux un espace en forme de cage (5) comportant des faces destinées à venir en contact avec l'os du fémur, la cage comportant au moins deux faces intérieures, planes ou sensiblement planes, à savoir une face (8) intérieure plane du côté antérieur de la partie la plus proximale du au moins un condyle et une face (10) intérieure plane du côté postérieur de la partie la plus proximale de la partie de trochlée, **caractérisée en ce que** la face (8) intérieure plane du côté antérieur de la partie la plus proximale du au moins un condyle, notamment du condyle latéral et/ou du condyle médial, est inclinée par rapport à l'axe mécanique du fémur, notamment d'un angle compris entre 1° et 5°, par exemple de 3°, en convergeant avec cet axe dans la direction proximale, et la face intérieure (8) plane du côté antérieur de la partie la plus proximale du au moins un condyle est inclinée par rapport à la face (10) intérieure plane la plus proximale du côté postérieur de la partie de trochlée d'un angle tel que ces deux faces convergent l'une vers l'autre dans la direction distale, c'est-à-dire que les deux faces sont convergentes, en section sagittale, vers un point se trouvant du côté du tibia.

2. Partie fémorale suivant la revendication 1, **caractérisée en ce que** le au moins un condyle comporte deux condyles respectivement médial et latéral, et les faces intérieures des deux condyles sont parallèles.

3. Partie fémorale suivant la revendication 1 ou 2, **caractérisée en ce que** la cage comporte aussi une face (9) intérieure plane de fond, disposée entre la face intérieure plane du côté antérieur de la partie la plus proximale du au moins un condyle et la face intérieure plane la plus proximale du côté antérieur de la partie de trochlée, la face de fond étant perpendiculaire à l'axe mécanique du fémur.

4. Partie fémorale suivant l'une des revendications 1 à 3, **caractérisée en ce que** l'angle de convergence proximale est compris entre 1 et 4°, notamment est égal à 3°.

5. Prothèse totale de genou comportant une partie fémorale suivant l'une des revendications précédentes et une partie tibiale constituée d'un plateau tibial et d'un ménisque.
